# EUROPEAN PATENT APPLICATION

(11) **EP 1 426 957 A2**
(43) Date of publication of application: **09.06.2004**
(21) Application number: 03257395.8
(22) Date of filing: 24.11.2003
(51) Int. Cl.: G11B 20/12, G11B 5/008, G06F 3/06, G11B 27/036

(54) **Configuring a storage medium using a logical cylindrical recording format**

(30) Priority: 22.11.2002 US 302786
(71) Applicant: QUANTUM CORPORATION, San Jose, California 95110 (US)
(72) Inventor: Saliba, George, Northborough, Massachusetts 01532 (US); King, Christopher P., Brimfield, Massachusetts 01010 (US)
(74) Representative: Charig, Raymond Julian

(57) **Abstract**

A data storage medium is partitioned into a number of storage rings. Each storage ring has a defined size. One or more storage rings are grouped into one or more storage cylinders, where the defined size of each storage ring in a storage cylinder is the same and the number of storage rings in a storage cylinder is set to a predetermined number.

## Description

### CROSS-REFERENCE TO RELATED APPLICATION

The present application is a continuation-in-part application of U.S. Patent Application Serial No. 09/577,637, entitled DATA STORAGE DEVICES FOR LARGE SIZE DATA STRUCTURES, filed on May 24, 2000, which claims the benefit of U.S. Provisional Application Serial No. 60/135,626, filed on May 24, 1999, both of which are incorporated in their entirety herein by reference.

### BACKGROUND

### 1. Field of the Invention

The present application generally relates to storing data on a storage medium, and more particularly to configuring a storage medium using a logical cylindrical recording (LCR) format.

### 2. Related Art

Data can be stored in various types of storage media in various formats. For example, in a conventional format for storing data on a hard disk, the surface of a platter of the hard disk is divided into a number of tracks and sectors. A track corresponds to a single circular portion of the platter. A sector corresponds to a portion of a track. The data to be stored on the hard disk is divided into pieces such that a single piece of the data can be stored within a single sector of the hard disk. The logical connection between the pieces of the data and the corresponding sectors of the hard disk is maintained in a table. Initially, the pieces of the data may be stored in sectors that are close together on the hard disk. However, over time, as the data is repeatedly read, modified, and written to the hard disk, the sectors can be scattered over the hard disk. This effect is commonly known as fragmentation, which can produce access delays.

In a conventional format for storing data on a magnetic tape, data is written on the magnetic tape in a continuous stream to create a track that may extend lengthwise across the entire magnetic tape. Multiple files are typically stored on multiple tracks with each new file beginning where the previous file ended. Thus, new data or files are only appended at the end of previously written data or files. Thus, when data located in the middle of a magnetic tape is modified, the data typically has to be rewritten from that point on the magnetic tape to the end of the magnetic tape, which can produce access delays.

In another conventional format for storing data on a magnetic tape, data is written on the magnetic tape in a "serpentine" pattern to create parallel, bi-directional tracks. In still another conventional format for storing data on a magnetic tape, data is written on the magnetic tape in a "helical" pattern. In both of these conventional formats, new data or files are only appended at the end of the previously written data or files, which can produce access delays.

### SUMMARY

In one exemplary embodiment, a data storage medium is partitioned into a number of storage rings. Each storage ring has a defined size. One or more storage rings are grouped into one or more storage cylinders, where the defined size of each storage ring in a storage cylinder is the same and the number of storage rings in a storage cylinder is set to a predetermined number.

### BRIEF DESCRIPTION OF THE FIGURES

Fig. 1 depicts an exemplary host terminal connected to various storage devices;

Figs. 2-A to 2-C depict exemplary storage rings;

Figs. 3-A and 3-B depict exemplary storage cylinders;

Figs. 4-A to 4-C depict various exemplary configurations of storage cylinders;

Figs. 5-A and 5-B depict exemplary storage cylinders stored on a magnetic tape;

Figs. 6-A to 6-C depict exemplary storage cylinders and cylinder sets;

Fig. 7 depicts exemplary storage cylinders;

Fig. 8 depicts exemplary cylinder sets;

Fig. 9 depicts storage rings, storage cylinders, and cylinder sets on a magnetic tape;

Fig. 10 depicts an exemplary tape drive and an exemplary tape cartridge;

Fig. 11 depicts an exemplary tape cartridge loaded into an exemplary tape drive; and

Figs. 12-A and 12-B depict storage rings and a storage cylinder on platters of a hard drive.

### DETAILED DESCRIPTION

The following description sets forth numerous specific configurations, parameters, and the like. It should be recognized, however, that such description is not intended as a limitation on the scope of the present invention, but is instead provided to provide a better description of exemplary embodiments.

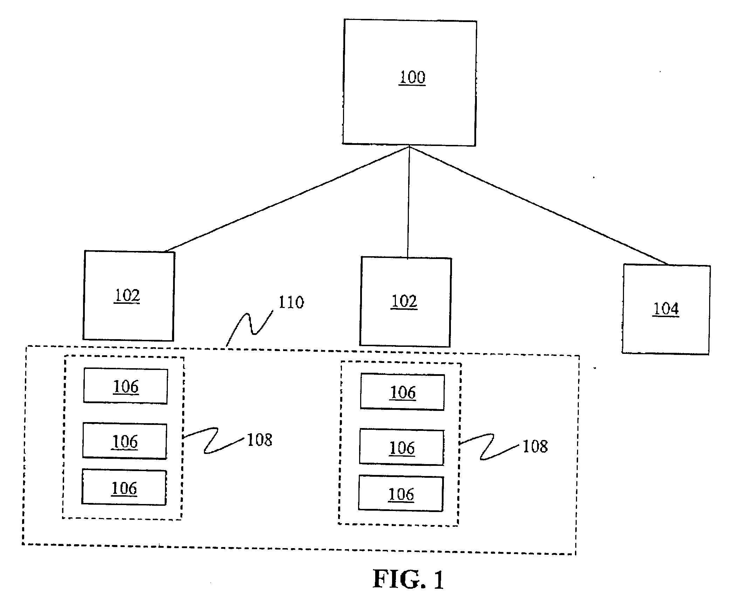

With reference to Fig. 1, a host terminal 100 is connected to tape drives 102 and a hard drive 104. Host terminal 100 can be any type of computer, such as a personal computer, a workstation, a server, and the like. Host terminal 100 can be connected to any number of tape drives 102 and/or hard drives 104. Additionally, any number of host terminals 100 can be connected to one or more tape drives 102 and/or hard drives 104.

In one exemplary embodiment, data is stored using a logical cylindrical recording (LCR) format on a data storage medium of a data storage device, such as a magnetic tape used in a tape drive 102 and/or one or more platters of a hard drive 104. In accordance with one aspect of the LCR format, the storage medium of a storage device is partitioned into one or more logical storage rings and storage cylinders.

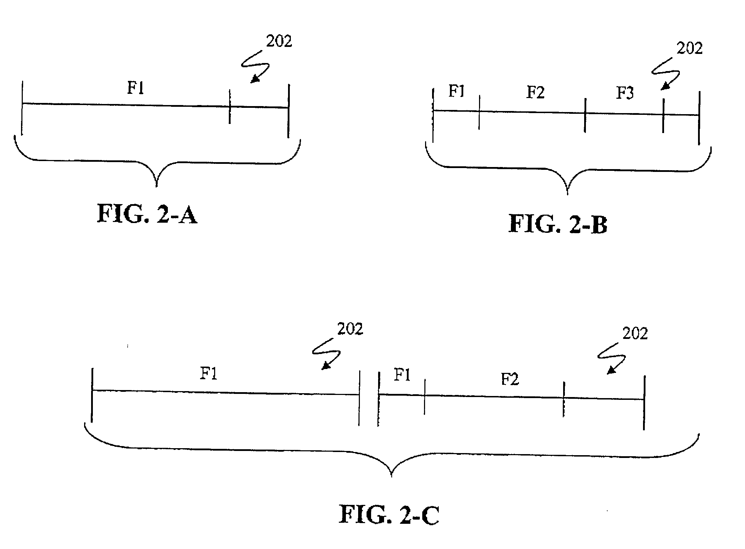

More specifically, with reference to Figs. 2-A to 2-C, a storage ring 202 has a defined length, which can store data corresponding to one file, multiple files, or a portion of a file. For example, Fig. 2-A depicts data corresponding to one file (F1) stored entirely within one storage ring 202. Fig. 2-B depicts data corresponding to multiple files (F1, F2, and F3) stored entirely within one storage ring 202. Fig. 2-C depicts data corresponding to one file (F1) stored in one storage ring 202 and a portion of another storage ring 202. The files stored on storage rings 202 can be uniform in length or varied in length (as depicted in Figs. 2-B and 2-C).

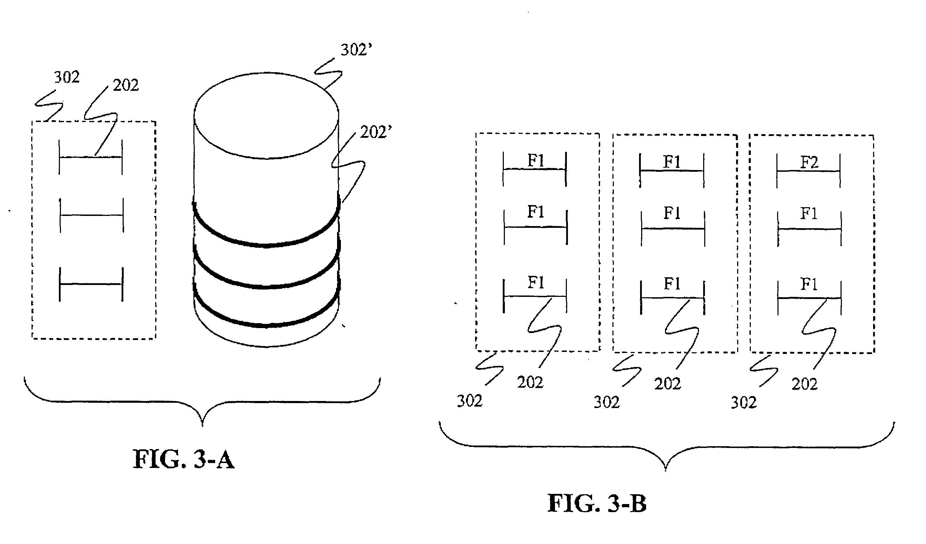

With reference to Fig. 3-A, one or more storage rings 202 are logically and physically grouped into a storage cylinder 302. All of the storage rings 202 in storage cylinder 302 have the same defined lengths. As depicted in Fig. 3-A, storage rings 202 and storage cylinder 302 can be conceptualized and depicted as physical rings 202' on a physical cylinder 302'.

Although any number of storage rings 202 can be grouped into a storage cylinder 302, in one exemplary embodiment, the number of storage rings 202 in storage cylinder 302 is set to a predetermined number. For example, for a particular storage medium that is used in a particular storage device, a designer of the particular storage device and/or a host connected to the particular storage device can set the number of storage rings in a storage cylinder (i.e., the predetermined number of storage rings) for the particular storage medium in the particular storage device. Thus, in one exemplary embodiment, all of the storage cylinders in the storage medium of the storage device have the same number of storage rings.

As noted above, a storage ring can store data corresponding to one file, multiple files, or a portion of a file. Similarly, a storage cylinder can store data corresponding to one file, multiple files, or a portion of a file. For example, Fig. 3-B depicts data corresponding to a large file (F1) stored in several storage cylinders 302.

In one exemplary embodiment, the size of a storage ring is about 1 kilobytes to about 100 kilobytes, and preferably about 5 kilobytes to about 10 kilobytes or about 6 kilobytes to about 12 kilobytes. The size of a storage cylinder is about 1 megabyte to about 100 megabytes.

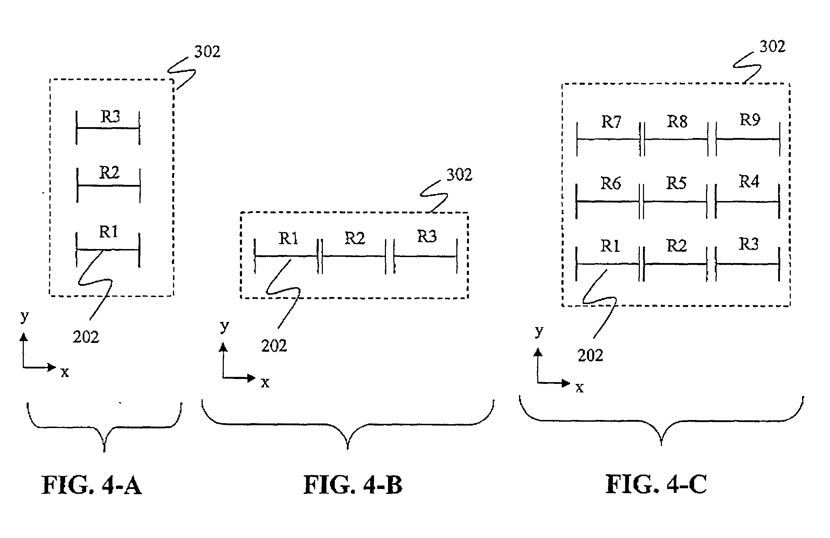

In accordance with one aspect of the LCR format, a storage cylinder has a dimension defined by the number and arrangement of the storage rings in the storage cylinder. For example, Figs. 4-A, 4-B, and 4-C depict exemplary storage cylinders of varying dimensions. More specifically, Fig. 4-A depicts a storage cylinder 302 with a (1, 3) dimension, meaning 1 storage ring in the x dimension and 3 storage rings in the y dimension. Fig. 4-B depicts a storage cylinder 302 with a (3, 1) dimension, meaning 3 storage rings in the x dimension and 1 storage ring in the y dimension. Fig. 4-C depicts a storage cylinder 302 with a (3, 3) dimension, meaning 3 storage rings in the x dimension and 3 storage rings in the y dimension.

Although only 1 and 2-dimensional storage cylinders 302 are depicted in Figs. 4-A to 4-C, it should be recognized that a 3-dimensional storage cylinder, which can be used in holography, can also be defined by having a set of storage rings in the x, y, and z dimensions. For example, the storage cylinder 302 depicted in Fig. 4-C can include one or more storage rings arranged along a third dimension, such as by overlaying the storage rings on top of the storage rings depicted in Fig. 4-C.

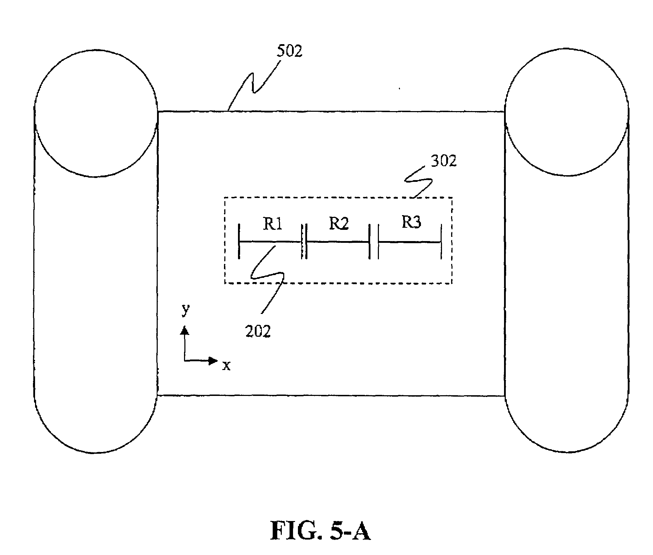

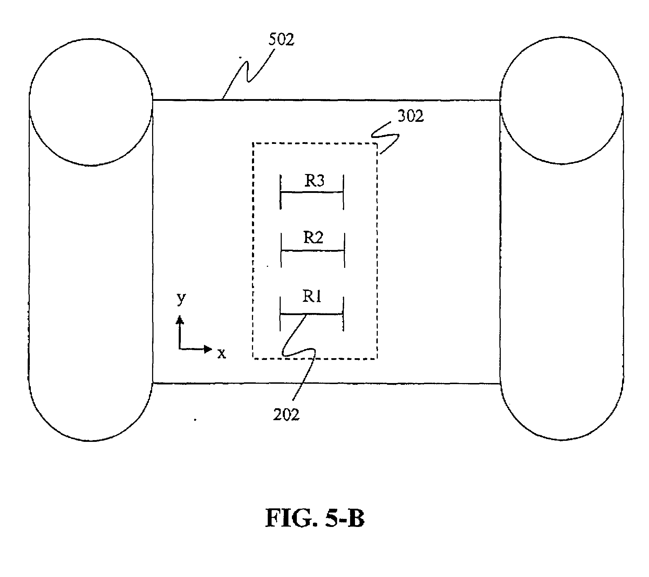

Although the dimensions of a storage cylinder can vary, it can be advantageous to match the dimensions of a storage cylinder to the characteristics of a storage medium. For example, with reference to Fig. 5-A, assume that a magnetic tape 502 in a tape cartridge of a tape drive is used as the storage medium. If data can be accessed on magnetic tape 502 more quickly in the longitudinal dimension of the magnetic tape (corresponding to the x-axis in Fig. 5-A) than the lateral dimension (corresponding to the y-axis in Fig. 5-A), storage cylinder 302 may be dimensioned with a greater number of storage rings in the longitudinal dimension than in the lateral dimension. With reference to Fig. 5-B, if data can be accessed on magnetic tape 502 more quickly in the lateral dimension of the magnetic tape (corresponding to the y-axis in Fig. 5-B) than the longitudinal dimension (corresponding to the x-axis in Fig. 5-B), storage cylinder 302 may be dimensioned with a greater number of storage rings in the lateral dimension than in the longitudinal dimension. In this manner, storage cylinder 302 can be more quickly accessed on magnetic tape 502.

In accordance with one aspect of the LCR format, as depicted in Figs. 4-A to 4-C, storage rings 202 within each storage cylinder 302 are written in order. Thus, for a particular storage ring, the logical address and the physical address of that particular storage ring are the same.

As depicted in Fig. 4-C, each storage ring 202 within storage cylinder 302 may be assigned a ring number (i.e., R1, R2, ..., R9), which is unique within storage cylinder 302. The ring number assigned to storage rings 202 can be a sequence of ordered numbers, such as 1, 2, 3, ..., 9. Alternatively, when storage cylinder 3 02 includes storage rings 202 extending in two dimensions, row and column numbers can be used as the ring numbers- It should be recognized, however, that various numbering schemes might be used to uniquely identify storage rings 202 within storage cylinder 302.

In Fig. 4-C, storage rings 202 are depicted as being written in what is known as a "serpentine" pattern. More specifically, the first row of storage rings 202 (i.e., R1, R2, and R3) are depicted as being written from left to right in Fig. 4-C. The second row of storage rings 202 (i.e., R4, R5, R6) are depicted as being written from right to left in Fig. 4-C. The third row of storage rings 202 (i.e., R7, R8, and R9) are depicted as being written in from left to right in Fig. 4-C. It should be recognized, however, that storage rings 202 can be written in a linear pattern. Thus, the second row of storage rings 202 (i.e., R4, R5, and R6) would be written from left to right. Thus, it should be recognized that storage rings 202 can be read in a serpentine or linear pattern. However, storage rings 202 should be read in the same pattern as they are written.

In one exemplary embodiment, the ring number is written at the end of a storage ring. Thus, the associating between a logical number for the storage ring and the physical location of the ring on the storage medium does not need to be stored, such as in a directory. Instead, the ring number of a storage ring can be determined as the storage medium is read. It should be noted, however, that the ring number can be written at any location of a storage ring.

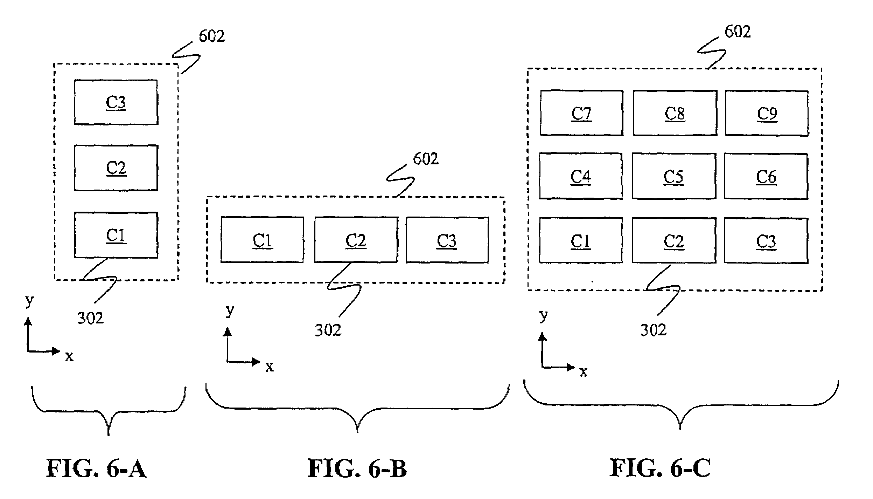

As noted above, one or more storage cylinders may be stored on a storage medium. Similar to storage rings, any number of storage cylinders may be arranged in any number of dimensions on the storage medium. For example, Fig. 6-A depicts storage cylinders 302 arranged in the y dimension. Fig. 6-B depicts storage cylinders 302 arranged in the x dimension. Fig. 6-C depicts storage cylinders 302 arranged in both the x and y dimensions. It should be noted that storage cylinders can also be arranged along a third dimension (i.e., the z dimension), such as by overlaying the storage cylinders on top of the storage cylinders depicted in Fig. 6-C.

Similar to storage rings, the number and arrangement of storage cylinders can be determined based on the characteristics of the storage medium. For example, if the storage medium is a magnetic tape and data can be accessed on the magnetic tape more quickly in the lateral dimension of the magnetic tape than the longitudinal dimension, a greater number of storage cylinders may be arranged along the lateral dimension than the longitudinal dimension. If data can be accessed on the magnetic tape more quickly in the longitudinal dimension of the magnetic tape than the lateral dimension, a greater number of storage cylinders may be arranged along the longitudinal dimension than the lateral dimension.

Also, similar to storage rings, storage cylinder can be written in order. With reference to Fig. 6-C, each storage cylinder may be assigned a cylinder number (i.e., C1, C2, ..., and C9). Similar to ring numbers, the cylinder numbers can be a sequence of ordered numbers, such as 1, 2, ..., and 9. Alternatively, when storage cylinders 302 are arranged in two dimensions, row and column numbers can be used as cylinder numbers. It should be recognized that various numbering schemes can be used to uniquely identify storage cylinders 302.

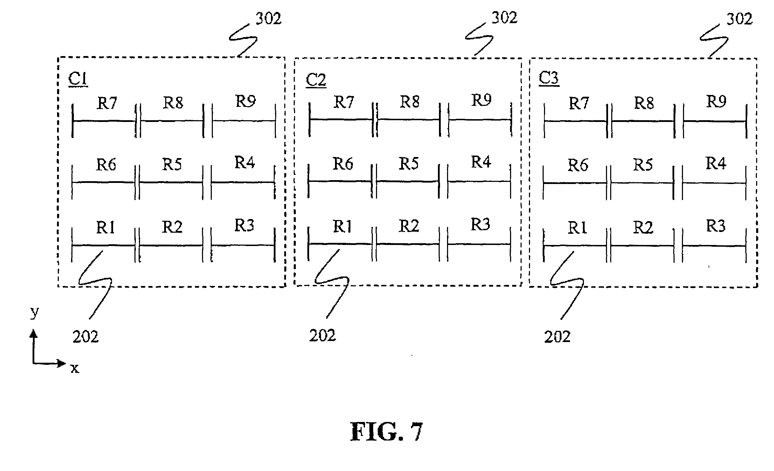

With reference to Fig. 7, when multiple storage cylinders 302 are assigned unique cylinder numbers, the numbers assigned to the group of storage rings 202 within each storage cylinder 302 can be reused. It should be recognized, however, that the numbers assigned to storage rings 202 can be unique as between multiple storage cylinders 302.

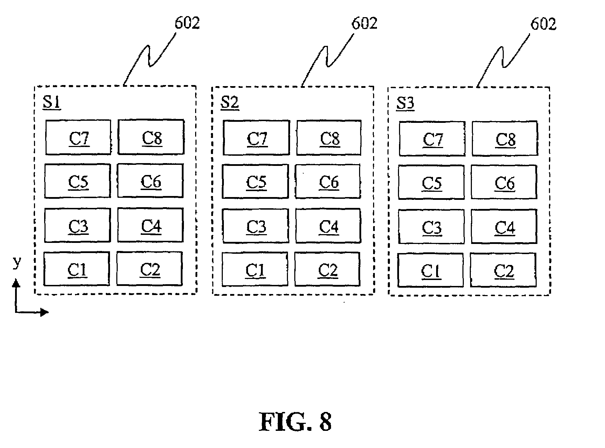

In accordance with one aspect of the LCR format, as depicted in Fig. 6-A to 6-C, one or more storage cylinders 302 can be organized as a cylinder set 602. Similar to storage rings and storage cylinders, cylinder sets can be written in order. With reference to Fig. 8, a cylinder set 602 may be assigned a cylinder-set number (i.e., S1, S2, and S3). Similar to ring numbers and cylinder numbers, the cylinder-set numbers can be a sequence of ordered numbers, such as 1, 2, and 3. Alternatively, when cylinder sets 602 are arranged in two dimensions, row and column numbers can be used as cylinder-set numbers. It should be recognized that various numbering schemes can be used to uniquely identify cylinder sets 602.

As depicted in Fig. 8, when multiple cylinder sets 602 are assigned unique cylinder-set numbers, the numbers assigned to the group of storage cylinders within each cylinder set 602 can be reused. It should be recognized, however, that the numbers assigned to the storage cylinders can be unique as between multiple cylinder sets 602.

In accordance with one aspect of the LCR format, the location of a set of data, such as data corresponding to a particular file, within a storage medium can be specified using a cylinder number, a ring number, and a location within the storage ring. The location within the storage ring can be determined based on a start address and the length of the data. Alternatively, the location within the storage ring can be determined based on a start address and an end address of the data

For example, with reference to Fig. 1, when the storage medium is a magnetic tape in a tape cartridge 106 of a tape drive 102, the mapping of ring numbers to storage cylinders may be stored in a directory by tape drive 102. Typically, to access data on tape drive 102, host terminal 100 transmits an object number to tape drive 102. Based on the object number, tape drive 102 can determine the cylinder number, the ring number, and the location within the storage ring corresponding to the object number.

As noted earlier, with reference to Fig. 4-A, a storage cylinder 302 can include any number of storage rings. However, in accordance with one aspect of the LCR format, the number of storage rings within a storage cylinder 302 is set to a predetermined number. For example, if a storage device has been configured to use storage cylinder 302 with 3 storage rings 202, then the number of storage rings 202 in storage cylinder 302 remains set at 3 unless the storage device is re-configured. Thus, if storage rings 202 in storage cylinder 302 are full, then data is written to another storage cylinder rather than adding an additional storage ring to storage cylinder 302- Thus, storage cylinder 302 is a self-contained structure and is independent of another storage cylinder.

In one exemplary embodiment, the size of the storage rings and dimensions of storage cylinders, which includes the predetermined number of storage rings in a storage cylinder, that are to be used in a storage device can be set by the designer of the storage device. For example, with reference to Fig. 1, the designer of tape drive 102 and/or hard drive 104, which are configured to use an LCR format, can determine and set the size of the storage rings and dimensions of the storage cylinders, including the predetermined number of storage rings in a storage cylinder, to use in tape drive 102, more specifically tape cartridges 106, and/or hard drive 104. Alternatively, in another exemplary embodiment, host terminal 100 can be configured to set the size of the storage rings and dimensions of storage cylinders, including the predetermined number of storage rings in a storage cylinder, to use in tape drive 102 and/or hard drive 104.

The size of the storage rings, the dimensions of the storage cylinders, and the number of storage cylinders are stored in a directory for a storage device, such as a tape cartridge of a tape drive or a hard disk. In one exemplary embodiment, one or more directories for a storage medium may be stored on the storage medium of the storage device. For example, in a tape cartridge, one or more directories for the tape cartridge can be stored at the beginning of the magnetic tape. When the tape cartridge is loaded into the tape drive, the one or more directories are then read into memory, for example by host computer 100. While the tape cartridge is in use, any changes to the one or more directories are made in memory. Before removing the tape cartridge from the tape drive, the one or more directories are written back to the beginning of the magnetic tape from memory. Alternatively, in another exemplary embodiment, the one or more directories are stored on a chip on the storage device.

In accordance with one aspect of the LCR format, a storage ring within a storage cylinder is modified by accessing the entire storage cylinder. For example, with reference to Fig. 4-A, a storage ring 202, such as R1, in storage cylinder 302 is modified by loading storage cylinder 302 from the storage medium into memory, such as a Random Access Memory (RAM) in host terminal 100 (Fig. 1), modifying R2 in memory, then writing the modified storage cylinder 302 from memory back into the storage medium.

With reference to Fig. 1, if the storage cylinder was initially on a tape cartridge 106 of tape drive 102, host terminal 100 would load the storage cylinder from tape cartridge 106 into memory, such as a Random Access Memory (RAM) in host terminal 100, modify the appropriate storage ring or rings in the storage cylinder, then write the entire modified storage cylinder back into tape cartridge 106. Thus, the capacity of the memory device into which a storage cylinder is to be loaded should be a factor in determining the size of the storage rings and dimension of a storage cylinder. More specifically, the storage capacity of the memory device should be the same or greater than the total amount of data to be stored in a single storage cylinder.

As noted earlier, one or more storage cylinders can be grouped as a cylinder set. While a storage cylinder is less than or equal in size as the available capacity of the memory, a cylinder set can be configured to be greater in size than the memory.

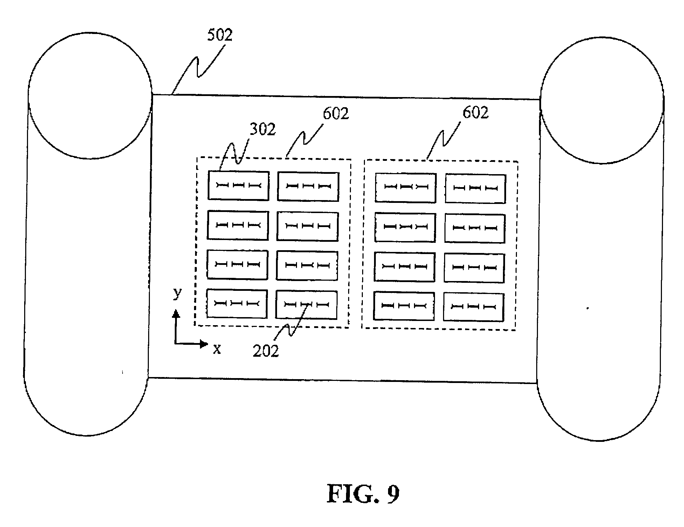

As noted above, a magnetic tape in a tape cartridge of a tape drive can be used as the storage medium. For example, in one exemplary embodiment, with reference to Fig. 9, magnetic tape 502 includes a plurality of storage rings 202, each storage ring corresponding to a logical and a physical partition of magnetic tape 502. In the exemplary embodiment depicted in Fig. 9, storage rings 202 are grouped together as storage cylinders 302, which are in turn grouped together as cylinder sets 602. As described above, each storage ring has a defined size, and the defined sizes of the storage rings within a storage cylinder are the same. Also, the number of storage rings 202 in a storage cylinder 302 is set to a predetermined number of storage rings, which is 3 in the exemplary embodiment depicted in Fig. 9. Although storage cylinders 302 in a cylinder set 602 are depicted as arranged in a matrix of 2 columns and 4 rows, it should be recognized that any number of storage cylinders 302 can be stored in any number of columns and any number of rows.

For example, assume a tape cartridge holds about 2,000 feet of magnetic tape and the tape cartridge is to store about 100 gigabytes of data. In one exemplary embodiment, the magnetic tape of the tape cartridge can be partitioned into 100,000,000 cylinder rings. Each cylinder ring having a defined length of about 10 kilobytes. In this exemplary embodiment, the predetermined number of storage rings in a storage cylinder is 1,000. Thus, 1,000 cylinder rings are grouped together as a storage cylinder for a total of 10,000 storage cylinders in the tape cartridge. Additionally, 1,000 storage cylinders are grouped together as a cylinder set for a total of 10 cylinder sets on the magnetic tape.

In another example, assume a tape cartridge holds about 2,000 feet of magnetic tape and the tape cartridge is to store about 1,000 gigabytes of data. In one exemplary embodiment, the magnetic tape of the tape cartridge can be partitioned into 100,000,00 cylinder rings. Each cylinder ring having a defined length of about 100 kilobytes. In this exemplary embodiment, the predetermined number of storage rings in a storage cylinder is again 1,000. Thus, 1,000 cylinder rings are grouped together as a storage cylinder for a total of 10,000 storage cylinders in the tape cartridge. Additionally, 1,000 storage cylinders are grouped together as a cylinder set for a total of 10 cylinder sets on the magnetic tape.

In accordance with one aspect of the LCR format, when the storage medium is a magnetic tape, the set of storage cylinders on a single tape cartridge is referred to as a cylinder module. Thus, with reference to Fig. 1, each tape cartridge 106 is a cylinder module. Additionally, a set of tape cartridges 106, such as in a library of tape cartridges, is referred to as a storage unit. Furthermore, a collection of multiple sets or libraries of tape cartridges 106 is referred to as a storage group. Thus, Fig. 1 depicts two storage units 108 and one storage group 110.

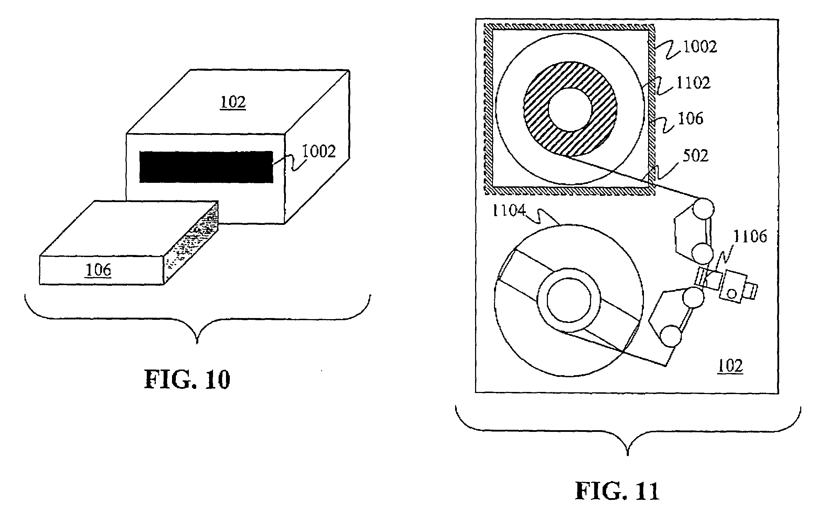

With reference to Fig. 10, a tape drive 102 is depicted having a receptacle 1002 configured to receive a tape cartridge 106. With reference to Fig. 11, tape cartridge 106 having a supply reel 1102 of magnetic tape 502 is depicted after being loaded into tape drive 102. Magnetic tape 502 includes data stored in accordance with the LCR format described herein. As depicted in Fig. 11, tape drive 102 includes a take-up reel 1104, which receives magnetic tape 502 from supply reel 1102, and a magnetic head 1106, which accesses (reads/writes) data on magnetic tape 502.

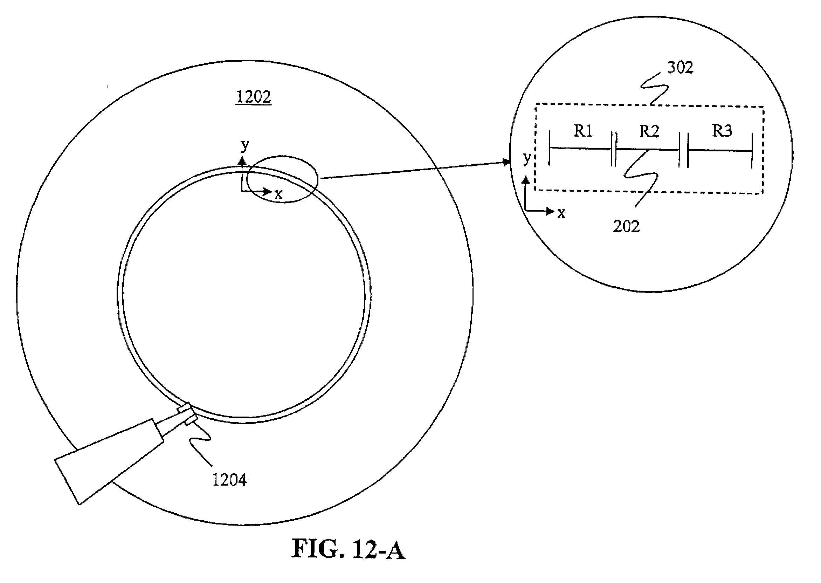

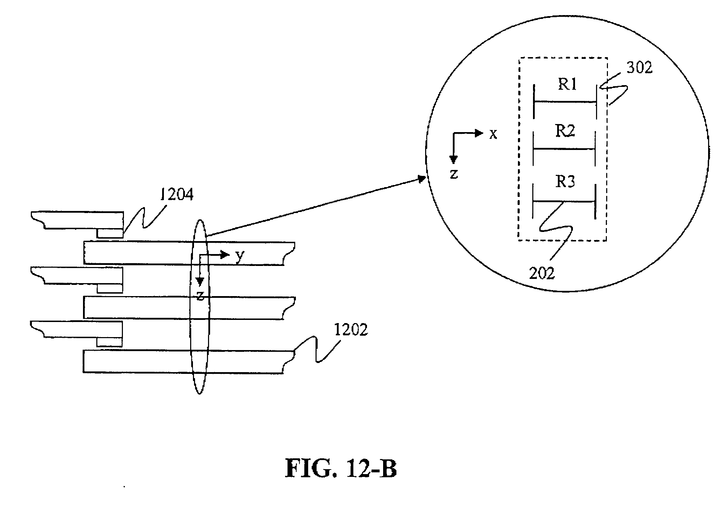

In another exemplary embodiment, a platter of a hard drive can be used as a storage medium. With reference to Fig. 12-A, an enlarged portion of a track on a platter 1202 of a hard drive is depicted. The track on platter 1202 is read by read/write head 1204. With reference to Fig. 12-B, the hard drive can include any number of platters 1202 with any number of read/write heads 1204.

As depicted in Fig. 12-A, storage rings 202 can be arranged extending along a track on platter 1202 (indicated as the x dimension in Fig. 12-A) and/or grouped as storage cylinder 302 extending along a track on platter 1202. Storage rings 202 can be arranged extending radially between multiple concentric tracks on platter 1202 (indicated as the y dimension in Fig. 12-B) and/or grouped as storage cylinder 302 extending radially between multiple concentric tracks on platter 1202. With reference to Fig. 12-B, storage rings 202 can be arranged extending between multiple platters 1202 (indicated as the z-dimension in Fig. 12-B) and/or grouped as storage cylinder 302 extending between multiple platters 1202. Additionally, storage rings 202 can be arranged extending along any combination of dimensions and/or grouped as storage cylinder 302 extending along any combination of dimensions. Although not depicted, one or more storage cylinders 302 can be grouped together as a cylinder set in the same manner.

In accordance with one aspect of the LCR format, one or more storage rings within a storage cylinder can be designated as data storage rings, which can be used for storing data, and one or more storage rings within the storage cylinder can be designated as redundancy storage rings, which can be used for error correction when one or more of the data storage rings in the storage cylinder becomes defective. In one exemplary embodiment, one or more redundancy storage rings can be designated as error correction code (ECC) storage rings, which can be used to correct data that has been stored in defective storage rings using any known data correction techniques, such as using Reed-Solomon codes. Additionally, one or more redundancy storage rings can be designated as alternate rings, which can be used to store data that was to be stored in defective storage rings.

In one exemplary embodiment, the data storage rings and redundancy storage rings can be arranged in separate locations within a storage cylinder. Alternatively, the data storage rings and redundancy storage rings can be intermingled within the storage cylinder.

With reference to Fig. 4-C, for the sake of example, assume that at least one storage ring 202, such as R1, has been designated as a data storage ring. Assume that at least one storage ring 202, such as R4, has been designated as an ECC storage ring. Also, assume that at least one storage ring 202, such as R5, has been designated as an alternate storage ring. Thus, in this example, R4 and R5 have been designated as redundancy storage rings.

In this example, if R1 becomes defective or determined to be defective during reading of data, then data stored in R1 can be corrected using R4, which has been designated as the ECC storage ring. Also, if R1 becomes defective or determined to be defective by reading after writing during recording of data, then data to be written to R1 can be written instead to R5.

Additionally, a defective storage ring, such as R1, can be indicated using any known error detection code and/or technique, such as using a cyclic redundancy checking (CRC) code. For example, when reading storage cylinder 302, a CRC code corresponding to R1 can be used to indicate that R1 is defective and ifRl is the first defective storage ring encountered, data is read from the first designated alternate ring (i.e., R5) rather than R1. Thus, one advantage of using an error detection code and/or technique to indicate the existence of a defective storage ring in combination with the use of redundancy storage rings is that the address of a defective storage ring may not need to be stored.

The number of storage rings in a storage cylinder designated as data storage rings and redundancy rings can be set based on a desired redundancy ratio. For example, for a storage cylinder with 1000 storage rings, a 25 percent redundancy ratio can be achieved by designating 750 storage rings as data storage rings and 250 storage rings as redundancy storage rings.

Additionally, the number of redundancy storage rings designated as ECC storage rings and alternate storage rings can be set based on a desired error correction ratio and a desired alternate ratio. For example, using the above example of a storage cylinder with 1000 storage rings with 750 data storage rings and 250 redundancy storage rings, an error correction ratio of 20 percent can be achieved by designating 200 of the redundancy storage rings as ECC storage rings. Thus, for every 4 data storage rings, there is at least 1 ECC storage ring. Also, an alternate ratio of 5 percent can be achieved by designating 50 of the redundancy storage rings as alternate rings. Thus, for every 15 data storage ring, there is at least 1 alternate storage ring.

In accordance with one aspect of the LCR format, if a storage cylinder has more defects than that can be handled by the redundancy storage rings, then that storage cylinder is a defective storage cylinder and an alternate storage cylinder is used. More specifically, data to be written to the defective storage cylinder is written instead to the alternate storage cylinder.

In one exemplary embodiment, the storage cylinder physically next to the defective storage cylinder may be used as an alternate storage cylinder. The data that was to be stored in the defective storage cylinder is stored in the alternate storage cylinder. The cylinder number of the defective storage cylinder may be stored in a directory for the storage device. Thus, when reading the data, the data that was to have been read from the defective storage cylinder is read from the storage cylinder next to the storage cylinder identified as being defective in the directory. As noted earlier, one or more directories can be stored on the storage medium of the storage device or on a chip on the storage device. One advantage to this embodiment is that access delay is reduced because the alternate storage cylinder is physically next to the defective storage cylinder. Another advantage to this embodiment is that the number of alternate storage cylinders used is based on the number of defective storage cylinders. Thus, the number of alternate storage cylinders is not fixed or pre-determined.

In another exemplary embodiment, one or more storage cylinders can be designated as alternate storage cylinders. The alternate storage cylinders can be physically located in one portion of the storage medium, such as at the end, or scattered throughout the storage medium. In this embodiment, the locations of the alternate storage cylinders and/or the association between the defective storage cylinders and the alternate storage cylinders may be stored in a directory.

Although exemplary embodiments have been described, various modifications can be made without departing from the spirit and/or scope of the present invention. Therefore, the present invention should not be construed as being limited to the specific forms shown in the drawings and described above.

## Claims

1. A method of configuring a data storage medium, comprising:
partitioning the data storage medium into a plurality of storage rings,
wherein each storage ring has a defined size; and
grouping one or more storage rings into one or more storage cylinders,
wherein the defined size of each storage ring in a storage cylinder is the same, and
wherein the number of storage rings in a storage cylinder is set to a predetermined number.

2. The method of claim 1, wherein a storage cylinder has a dimension defined by the number and arrangement of the one or more storage rings in the storage cylinder.

3. The method of claim 2, wherein a storage cylinder includes one or more storage rings arranged along a first dimension and one or more storage rings arranged along a second dimension.

4. The method of claim 3, wherein the storage cylinder includes one or more storage rings arranged along a third dimension.

5. The method of claim 3, wherein the storage medium is used in a storage device, and wherein an access rate of the storage medium in the storage device is greater in a first dimension than a second dimension of the storage medium, and wherein the storage cylinder has a greater number of storage rings along the first dimension than the second dimension of the storage medium.

6. The method of claim 1 further comprising:
assigning a ring number to each storage ring; and
assigning a cylinder number to each storage cylinder,
wherein a location of a set of data within the storage medium is defined by the cylinder number, the ring number, and a location within a storage ring.

7. The method of claim 6, wherein the location within a storage ring is defined by a start address and a length of a file.

8. The method of claim 6, wherein the location within a storage ring is defined by a start address and an end address.

9. The method of claim 1 further comprising:
writing one or more storage cylinders in order within the storage medium,
wherein each storage cylinder has a logical address and a physical address, and
wherein for a particular storage cylinder, the logical and physical addresses of that particular storage cylinder are the same.

10. The method of claim 9 further comprising:
writing one or more storage rings in order within a storage cylinder,
wherein each storage ring has a logical address and a physical address, and
wherein for a particular storage ring, the logical and physical addresses of that particular storage ring are the same.

11. The method of claim 1 further comprising:
modifying a storage ring within a storage cylinder by:
reading the storage cylinder from the storage medium into a memory,
modifying the storage ring in the memory, and
writing the storage cylinder back into the storage medium.

12. The method of claim 1 further comprising:
storing a ring size, a cylinder dimension, and a number of storage cylinders in one or more directories.

13. The method of claim 1 further comprising:
grouping one or more storage cylinders into a cylinder set.

14. The method of claim 13 further comprising:
writing one or more cylinder sets in order within the storage medium,
wherein each cylinder set has a logical address and a physical address, and
wherein for a particular cylinder set, the logical and physical addresses of that particular cylinder set are the same;
writing one or more storage cylinders in order within a cylinder set,
wherein each storage cylinder has a logical address and a physical address, and
wherein for a particular storage cylinder, the logical and physical addresses of that particular storage cylinder are the same; and
writing one or more storage rings in order within a storage cylinder,
wherein each storage ring has a logical address and a physical address, and
wherein for a particular storage ring, the logical and physical addresses of that particular storage ring are the same.

15. The method of claim 13, wherein the one or more storage cylinders within a cylinder set have the same number of storage rings.

16. The method of claim 1, wherein the predetermined number of storage rings is set by a designer of a storage device that stores data on the data storage medium.

17. The method of claim 1, wherein the predetermined number of storage rings is set by a host terminal connected to a storage device that stores data on the data storage medium.

18. The method of claim 1, wherein the storage medium is a magnetic tape of a tape cartridge.

19. The method of claim 18, wherein a tape cartridge defines a cylinder module.

20. The method of claim 18, wherein the one or more storage cylinders on the magnetic tape have the same number of storage rings.

21. The method of claim 1, wherein the storage medium includes one or more platters of a hard disk.

22. A data storage medium, comprising:
a plurality of storage rings, each storage ring corresponding to a logical and a physical partition of the data storage medium,
wherein each storage ring has a defined size; and
at least one storage cylinder having one or more storage rings,
wherein the defined size of each storage ring in a storage cylinder is the same, and
wherein the number of storage rings in a storage cylinder is set to a predetermined number.

23. The data storage medium of claim 22, wherein a storage cylinder has a dimension defined by the number and arrangement of the one or more storage rings in the storage cylinder.

24. The data storage medium of claim 23, wherein a storage cylinder includes one or more storage rings arranged along a first dimension and one or more storage rings arranged along a second dimension.

25. The data storage medium of claim 24, wherein the storage cylinder includes one or more storage rings defined along a third dimension.

26. The data storage medium of claim 24, wherein the storage medium is used in a storage device, wherein an access rate of the storage medium in the storage device is greater in a first dimension than a second dimension of the storage medium, and wherein the storage cylinder has a greater number of storage rings along the first dimension than the second dimension of the storage medium.

27. The data storage medium of claim 22, wherein a location of a file within the storage medium is defined by a cylinder number assigned to each storage cylinder, a ring number assigned to each storage ring, and a location within a storage ring.

28. The data storage medium of claim 22, wherein the storage rings are written in order in the storage medium.

29. The data storage medium of claim 22, wherein a storage ring within a storage cylinder is modified by:
reading the storage cylinder from the storage medium into a memory,
modifying the storage ring in the memory, and
writing the storage cylinder back into the storage medium.

30. The data storage medium of claim 22, wherein the predetermined number of storage rings is set by a designer of a storage device that stores data on the data storage medium.

31. The data storage medium of claim 22, wherein the predetermined number of storage rings is set by a host terminal connected to a storage device that stores data on the data storage medium.

32. A magnetic tape, comprising:
a plurality of storage rings, each storage ring corresponding to a logical and a physical partition of the magnetic tape,
wherein each storage ring has a defined size; and
at least one storage cylinder having one or more storage rings,
wherein the defined size of each storage ring in a storage cylinder is the same, and
wherein the number of storage rings in a storage cylinder is set to a predetermined number.

33. The magnetic tape of claim 32, wherein a storage cylinder includes one or more storage rings arranged along a first dimension and one or more storage rings arranged along a second dimension.

34. The magnetic tape of claim 33, wherein the magnetic tape is used in a tape drive, and wherein an access rate of the magnetic tape in the tape drive is greater in a first dimension than a second dimension of the magnetic tape, and wherein the storage cylinder has a greater number of storage rings along the first dimension than the second dimension of the magnetic tape.

35. The magnetic tape of claim 32, wherein a storage ring within a storage cylinder is modified by:
reading the storage cylinder from the storage medium into a memory,
modifying the storage ring in the memory, and
writing the storage cylinder back into the storage medium.

36. The magnetic tape of claim 32, wherein the predetermined number of storage rings is set by a designer of a storage device that stores data on the magnetic tape.

37. The magnetic tape of claim 32, wherein the predetermined number of storage rings is set by a host terminal connected to a storage device that stores data on the magnetic tape.

38. A tape drive, comprising:
a receptacle configured to receive a magnetic tape, wherein the magnetic tape includes:
a plurality of storage rings, each storage ring corresponding to a logical and a physical partition of the magnetic tape,
wherein each storage ring has a defined size;
at least one storage cylinder having one or more storage rings,
wherein the defined size of each storage ring in a storage cylinder is the same, and
wherein the number of storage rings in a storage cylinder is set to a predetermined number; and
at least one magnetic head configured to access (read/write) data on the magnetic tape.

39. The tape drive of claim 38, wherein a storage cylinder includes one or more storage rings arranged along a first dimension and one or more storage rings arranged along a second dimension.

40. The tape drive of claim 39, wherein an access rate of the magnetic tape in the tape drive is greater in a first dimension than a second dimension of the magnetic tape, and wherein the storage cylinder has a greater number of storage rings along the first dimension than the second dimension of the magnetic tape.

41. The tape drive of claim 38, wherein a storage ring within a storage cylinder is modified by:
reading the storage cylinder from the storage medium into a memory,
modifying the storage ring in the memory, and
writing the storage cylinder back into the storage medium.

42. The method of claim 38, wherein the predetermined number of storage rings is set by a designer of the tape drive.

43. The method of claim 38, wherein the predetermined number of storage rings is set by a host terminal connected to the tape drive.
